(19) Europäisches Patentamt
European Patent Office
Office européen des brevets

(11) Veröffentlichungsnummer: **0 306 876 B1**

# (12) EUROPÄISCHE PATENTSCHRIFT

(45) Veröffentlichungstag der Patentschrift: **21.06.95**

(51) Int. Cl.⁶: **C07D 487/04**, //(C07D487/04, 239:00,231:00)

(21) Anmeldenummer: **88114433.1**

(22) Anmeldetag: **03.09.88**

Die Akte enthält technische Angaben, die nach dem Eingang der Anmeldung eingereicht wurden und die nicht in dieser Patentschrift enthalten sind.

## (54) Verfahren zur Herstellung von Pyrazolo [5,1-b] chinazolonen.

(30) Priorität: **11.09.87 DE 3730536**

(43) Veröffentlichungstag der Anmeldung:
**15.03.89 Patentblatt 89/11**

(45) Bekanntmachung des Hinweises auf die Patenterteilung:
**21.06.95 Patentblatt 95/25**

(84) Benannte Vertragsstaaten:
**CH DE FR GB IT LI**

(56) Entgegenhaltungen:
**EP-A- 0 015 065**

**ANGEWANDTE CHEMIE, 74. Jahr- gang 1962, Nr. 21 MENZEL et al. "Synthesen undReaktionen neuer ortho -kondensierter Pyrazoloverbindungen" Seiten 839-847**

(73) Patentinhaber: **BASF AKTIENGESELLSCHAFT**

**D-67056 Ludwigshafen (DE)**

(72) Erfinder: **Kanter, Hartmut, Dr.**
**Niedererdstrasse 105**
**D-6700 Ludwigshafen (DE)**
Erfinder: **Ort, Burkhard, Dr.**
**Waldstrasse 63**
**D-6706 Wachenheim (DE)**

EP 0 306 876 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein neues Verfahren zur Herstellung von Pyrazolo[5,1-b]chinazolonen durch Umsetzung von Isatosäureanhydriden mit Pyrazolonen in einem inerten organischen Lösungsmittel bei einer Temperatur von 100 bis 180 °C unter gleichzeitiger azeotroper Destillation des bei der Umsetzung entstehenden Wassers.

Es ist bekannt, 2-Methylpyrazolo[5,1-b]chinazolon durch Umsetzung von Isatosäureanhydrid mit 3-Methylpyrazol-5-on in der Schmelze oder in einem hochsiedenden Lösungsmittel bei einer Temperatur von 200 bis 250 °C durchzuführen (Angew. Chem., Band 74, S. 839 (1962)).

Aus J. Heterocycl. Chem., Band 18, S. 117 (1981), und J. Med. Chem., Band 24, S. 735 (1981), ist die Herstellung weiterer Pyrazolo[5,1-b]chinazolone bekannt. Als Ausgangsprodukte dienen ebenfalls Isatosäureanhydride und Pyrazolone. Die Umsetzung findet dabei in N,N-Dimethylformamid als Lösungsmittel in Gegenwart von Natriumhydrid als Base bei einer Temperatur von -10 bis 0 °C statt.

Beide Verfahrensweisen sind im technischen Maßstab nicht ohne weiteres durchführbar. Zum einen bedarf es besonderer Maßnahmen, um die Umsetzung bei sehr hoher Temperatur in einer Schmelze oder in einem hochsiedenden Lösungsmittel vornehmen zu können; zum anderen ist die Verwendung von Natriumhyrid als Base nicht unproblematisch und verlangt einen hohen Sicherheitsaufwand.

Aufgabe der vorliegenden Erfindung war es, ein neues Verfahren zur Herstellung von Pyrazolo[5,1-b]-chinazolonen bereitzustellen, das von technisch gut zugänglichen Ausgangsprodukten ausgehen und das die Zielprodukte ohne großen technischen Aufwand und in guter Ausbeute liefern sollte.

Es wurde nun gefunden, daß die Herstellung von Pyrazolo[5,1-b]chinazolonen der Formel I

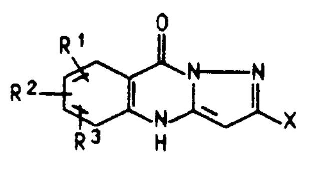

(I),

in der $R^1$, $R^2$ und $R^3$ gleich oder verschieden sind und unabhängig voneinander jeweils Wasserstoff, Halogen, $C_1$-$C_4$-Alkoxy, Nitro, $C_1$-$C_4$-Alkyl, Trifluormethyl, Carboxy, Cyano, Carbamoyl, $C_1$-$C_4$-Mono- oder Dialkylcarbamoyl, Phenylcarbamoyl, Sulfamoyl, $C_1$-$C_4$-Mono- oder Dialkylsulfamoyl, Phenylsulfamoyl, Hydroxysulfonyl oder $C_1$-$C_4$-Alkanoylamino und X $C_1$-$C_4$-Alkyl oder gegebenenfalls substituiertes Phenyl bedeuten, durch Umsetzung von Isatosäureanhydriden der Formel II

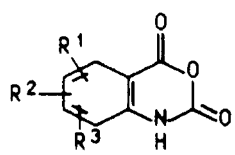

(II),

in der $R^1$, $R^2$ und $R^3$ jeweils die obengenannte Bedeutung besitzen, mit Pyrazolonen der Formel III

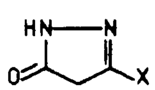

(III),

in der X die obengenannte Bedeutung besitzt, vorteilhaft gelingt, wenn man die Umsetzung bei einer Temperatur von 100 bis 180 °C in einem inerten organischen Lösungsmittel unter gleichzeitiger azeotroper Destillation des bei der Umsetzung entstehenden Wassersdurchführt.

Alle in den obengenannten Formeln auftretenden Alkylgruppen können sowohl geradkettig als auch verzweigt sein.

Für den Fall, daß in den Formeln I und III substituierte Phenylgruppen auftreten, kommen als Substituenten z.B. $C_1$-$C_4$-Alkyl, $C_1$-$C_4$-Alkoxy, Halogen, insbesondere Fluor, Chlor oder Brom, Nitro oder Cyano in Betracht.

$R^1$, $R^2$, $R^3$ und X bedeuten z.B. Methyl, Ethyl, Propyl, Isopropyl, Butyl, Isobutyl oder sec-Butyl.

X bedeutet weiterhin z.B. Phenyl, 2-Methylphenyl, 4-Methylphenyl, 4-Ethylphenyl, 4-Propylphenyl, 4-Isopropylphenyl, 2,4-Dimethylphenyl, 2-Methoxyphenyl, 4-Ethoxyphenyl, 4-Isopropoxyphenyl, 4-Butoxyphenyl, 2,6-Dimethoxyphenyl, 4-Fluorphenyl, 4-Chlorphenyl, 4-Bromphenyl, 2,4-Dichlorphenyl, 2- oder 3-Nitrophenyl oder 3- oder 4-Cyanophenyl.

$R^1$, $R^2$ und $R^3$ bedeuten weiterhin z.B. Fluor, Chlor, Brom, Methoxy, Ethoxy, Propoxy, Isopropoxy, Butoxy, Isobutoxy, sec-Butoxy, Mono- oder Dimethylcarbamoyl, Mono- oder Diethylcarbamoyl, Mono- oder Dipropylcarbamoyl, Mono- oder Diisopropylcarbamoyl, Mono- oder Dibutylcarbamoyl, N-Methyl-N-ethylcarbamoyl, Mono- oder Dimethylsulfamoyl, Mono- oder Diethylsulfamoyl, Mono- oder Dipropylsulfamoyl, Mono- oder Diisopropylsulfamoyl, Mono- oder Dibutylsulfamoyl, N-Methyl-N-ethylsulfamoyl, Formylamino, Acetylamino, Propionylamino, Butyrylamino oder Isobutyrylamino.

Bevorzugt ist das erfindungsgemäße Verfahren für solche Pyrazolo[5,1-b]chinazolone der Formel I, in der X Methyl oder Phenyl und $R^1$, $R^2$ und $R^3$ Wasserstoff, Nitro, Methoxy, Phenylsulfamoyl, Chlor oder Brom bedeuten.

Das erfindungsgemäße Verfahren wird in einem inerten organischen Lösungsmittel bei einer Temperatur von 100 bis 180 °C, vorzugsweise 120 bis 160 °C durchgeführt.

Im allgemeinen arbeitet man unter Normaldruck. In manchen Fällen kann es jedoch von Vorteil sein, die Umsetzung auch unter erhöhtem Druck (0 bis zu 5 bar) vorzunehmen.

Als inertes organisches Lösungsmittel dient insbesondere Xylol, Dichlorbenzol, Nitrobenzol oder Benzoesäuremethylester.

Man verwendet dabei in der Regel 3 bis 10 Gew.-Teile Lösungsmittel, bezogen auf 1 Gew.-Teil Isatosäureanhydrid II.

Isatosäureanhydrid II und Pyrazolon III werden üblicherweise im Molverhältnis 1,2:1 bis 0,8:1 eingesetzt.

Es besteht auch die Möglichkeit, das erfindungsgemäße Verfahren in Gegenwart von 1 bis 50 Gew.%, vorzugsweise 5 bis 30 Gew.% und insbesondere 10 bis 20 Gew.%, jeweils bezogen auf das Pyrazolon der Formel III, an wasserfreiem Natrium- oder Kaliumcarbonat durchzuführen.

Zweckmäßig wird das neue Verfahren so durchgeführt, daß man inertes Lösungsmittel und Pyrazolon III vorlegt, unter Rühren auf die erfindungsgemäße Temperatur erhitzt und Isatosäureanhydrid II einträgt. Nach beendeter Kohlendioxidentwicklung wird das Reaktionsgemisch gegebenenfalls mit Katalysator versetzt und im allgemeinen 1 bis 10 Stunden bei der erfindungsgemäßen Temperatur nachgerührt, wobei gleichzeitig das bei der Umsetzung entstehende Wasser aus dem Reaktionsgemisch azeotrop abdestilliert wird. Als Schleppmittel dient dabei das inerte Lösungsmittel.

Isatosäureanhydrid II und Pyrazolon III können auch als wäßrige Paste eingesetzt werden, wobei das Wasser zusammen mit dem Reaktionswasser aus dem Reaktionsgemisch azeotrop abdestilliert wird.

Die während der Reaktion abdestillierte Menge an Lösungsmittel kann gegebenenfalls in Form von frischem oder regeneriertem Lösungsmittel dem Reaktionsgemisch wieder zugeführt werden.

Nach beendeter Umsetzung, die über eine ringoffene Zwischenverbindung der Formel IV

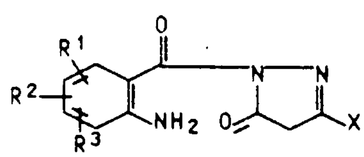

(IV),

in der $R^1$, $R^2$, $R^3$ und X jeweils die obengenannte Bedeutung besitzen, abläuft, wird das resultierende Pyrazolo[5,1-b]chinazolon der Formel I abgetrennt, mit dem jeweiligen Lösungsmittel, Methanol und warmem Wasser gewaschen und getrocknet.

Es ist auch möglich, das Lösungsmittel mittels Wasserdampfdestillation zu entfernen und das Pyrazolo-[5,1-b]chinazolon aus wäßriger Suspension zu isolieren. Verunreinigungen gehen dabei bei einem pH-Wert oberhalb von 7 in Lösung und können somit bei der Filtration entfernt werden.

Mittels des erfindungsgemäßen Verfahrens, das sowohl in kontinuierlicher als auch diskontinuierlicher Arbeitsweise durchgeführt werden kann, können die Pyrazolo[5,1-b]chinazolone ohne großen technischen Aufwand und in guten Ausbeuten erhalten werden. Die bei der Synthese eingesetzten Lösungsmittel können dabei im allgemeinen nach einer Regenerierung (Destillation) wiederverwendet werden.

Bei den Pyrazolo[5,1-b]chinazolonen handelt es sich um wertvolle Zwischenprodukte für die Synthese von Farbstoffen und Pigmenten.

Die folgenden Beispiele sollen die Erfindung näher erläutern.

Beispiel 1

100 g 3-Methylpyrazol-5-on wurden in 1000 g Xylol vorgelegt. Nach Erhitzen auf 115 °C wurden 200 g 5-Chlorisatosäureanhydrid langsam in das Gemisch eingetragen. Nach Abklingen der $CO_2$-Entwicklung wurde 20 Minuten nachgerührt, dann auf 140 °C erhitzt und solange bei dieser Temperatur gehalten, bis kein Reaktionswasser mehr abdestillierte. Danach wurde auf 70 °C abgekühlt, filtriert, zunächst mit 550 g Methanol, dann mit warmem Wasser gewaschen und getrocknet. Man erhielt 200 g eines farblosen Pulvers der Formel

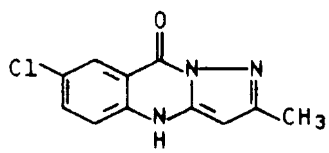

Beispiel 2

160 g 3-Phenylpyrazol-5-on wurden in 750 g Xylol gegeben und auf 115 °C erhitzt. Dann wurden 160 g Isatosäureanhydrid langsam in das Gemisch eingetragen. Nach Beendigung der $CO_2$-Entwicklung ließ man 20 Minuten nachrühren, erwärmte auf 140 °C und hielt diese Temperatur 6 Stunden lang. Danach kühlte man auf 95 °C ab, verdünnte mit 1000 ml Wasser und destillierte das Lösungsmittel durch Wasserdampfdestillation ab. Anschließend verdünnte man mit 2000 ml Wasser, versetzte mit 40 g Natronlauge (50 Gew.%) und rührte 1 Stunde nach. Dann wurde filtriert, mit Wasser neutral gewaschen und getrocknet. Man erhielt 220 g eines gelblichen Pulvers der Formel

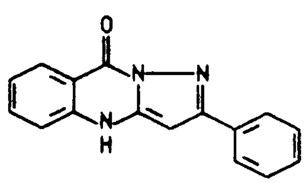

Beispiel 3

100 g 3-Methylpyrazol-5-on und 200 g 6-Chlorisatosäureanhydrid wurden in 700 g Nitrobenzol 4 Stunden auf 150 °C erhitzt. Man kühlte dann auf 80 °C ab und filtrierte das Reaktionsgemisch. Der Filterrückstand wurde mit 500 g Methanol und daran anschließend mit 3000 ml warmem Wasser gewaschen und getrocknet. Man erhielt 195 g eines farblosen Pulvers der Formel

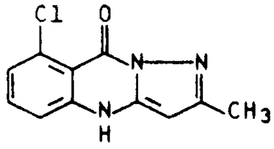

Beispiel 4

50 g 3-Methylpyrazol-5-on wurden in 350 g Xylol vorgelegt und auf 115 °C erhitzt. Bei dieser Temperatur trug man 80 g Isatosäureanhydrid langsam ein und rührte nach bis die $CO_2$-Entwicklung beendet war. Dann wurden 10 g wasserfreies Natriumcarbonat zugegeben, auf 135 °C erhitzt und solange nachgerührt, bis kein Reaktionswasser mehr überging. Man kühlte auf 100 °C ab, gab 200 ml Wasser zu und trieb Xylol mittels Wasserdampf aus. Anschließend wurde die wäßrige Suspension auf 70 °C abgekühlt

und mit 5 g Schwefelsäure (96 Gew.%) ein pH-Wert von 7 bis 7,5 eingestellt. Dann wurde abfiltriert, bis zu einem farblosen Ablauf mit warmem Wasser gewaschen und getrocknet. Man erhielt 85 g eines Produkts der Formel

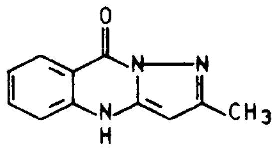

Beispiel 5

100 g 3-Methylpyrazol-5-on und 200 g 4-Chlorisatosäureanhydrid wurden in 800 g Dichlorbenzol 3 Stunden auf 155 °C erhitzt. Man kühlte auf 80 °C ab. filtrierte, wusch mit 500 g Methanol, dann mit 3000 ml warmem Wasser und trocknete. Man erhielt 180 g eines farblosen Pulvers der Formel

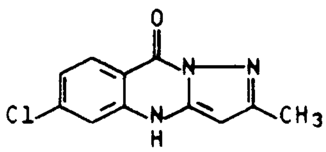

Beispiel 6

200 g 3-Methylpyrazol-5-on wurden in 1500 g Benzoesäuremethylester vorgelegt, unter Rühren auf 115 °C erhitzt und dann mit 300 g 3,5-Dichlorisatosäureanhydrid langsam versetzt. Unter Abdestillieren des Reaktionswassers wurde anschließend 3 Stunden bei 170 °C nachgerührt. Nach Abkühlen auf 90 °C wurde filtriert, mit 1000 g Methanol und 3000 ml warmem Wasser gewaschen und getrocknet. Man erhielt 250 g eines hellen Pulvers der Formel

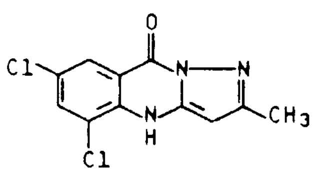

Die in der folgenden Tabelle aufgeführten Verbindungen wurden in analoger Weise erhalten. Reaktionsbedingungen und Ausbeute sind jeweils angegeben.

| Beispiel Nr. | Formel | Lösungsmittel | Temp. [°C] | Ausbeute [%] |
|---|---|---|---|---|
| 7 | 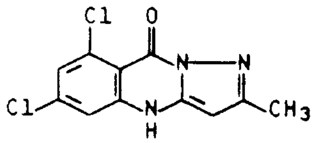 | Nitrobenzol | 160 | 73 |
| 8 | 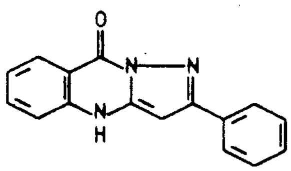 | Nitrobenzol | 165 | 85 |
| 9 | 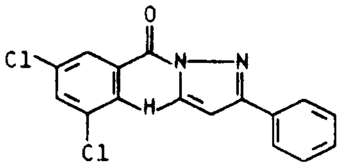 | Dichlorbenzol | 170 | 82 |

| Beispiel Nr. | Formel | Lösungsmittel | Temp. [°C] | Ausbeute [%] |
|---|---|---|---|---|
| 10 | 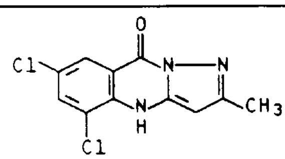 | Nitrobenzol | 170 | 84 |
| 11 | 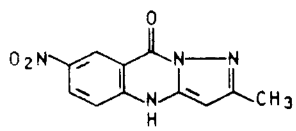 | Benzoesäuremethylester | 160 | 75 |
| 12 | 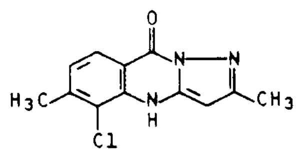 | Nitrobenzol | 160 | 78 |
| 13 | 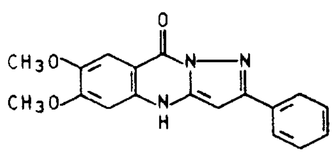 | Nitrobenzol | 160 | 84 |
| 14 | 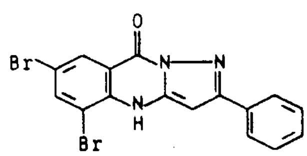 | Benzoesäuremethylester | 160 | 89 |
| 15 | 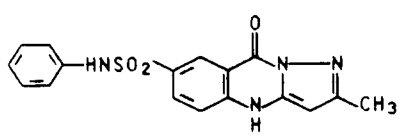 | Xylol | 140 | 87 |
| 16 | 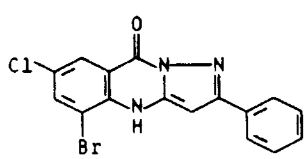 | Nitrobenzol | 170 | 83 |
| 17 | 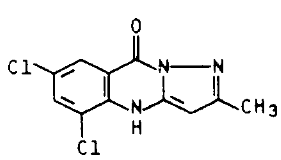 | Nitrobenzol | 170 | 85 |
| 18 | 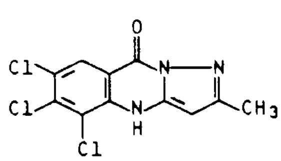 | Nitrobenzol | 170 | 75 |

## Patentansprüche

1. Verfahren zur Herstellung von Pyrazolo[5,1-b]chinazolonen der Formel I

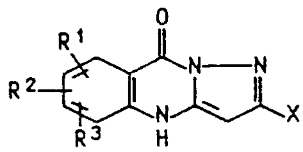

(I),

in der $R^1$, $R^2$ und $R^3$ gleich oder verschieden sind und unabhängig voneinander jeweils Wasserstoff, Halogen, $C_1$-$C_4$-Alkoxy, Nitro, $C_1$-$C_4$-Alkyl, Trifluormethyl, Carboxy, Cyano, Carbamoyl, $C_1$-$C_4$-Mono- oder Dialkylcarbamoyl, Phenylcarbamoyl, Sulfamoyl, $C_1$-$C_4$-Mono- oder Dialkylsulfamoyl, Phenylsulfamoyl, Hydroxysulfonyl oder $C_1$-$C_4$-Alkanoylamino und X $C_1$-$C_4$-Alkyl oder gegebenenfalls durch $C_1$-$C_4$-Alkyl, $C_1$-$C_4$-Alkoxy, Halogen, Nitro oder Cyano substituiertes Phenyl bedeuten, durch Umsetzung von Isatosäureanhydriden der Formel II

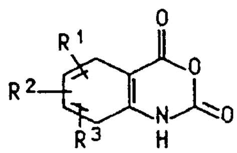

(II),

in der $R^1$, $R^2$ und $R^3$ jeweils die obengenannte Bedeutung besitzen, mit Pyrazolonen der Formel III

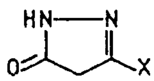

(III),

in der X die obengenannte Bedeutung besitzt, dadurch gekennzeichnet, daß man die Umsetzung bei einer Temperatur von 100 bis 180 °C in einem inerten organischen Lösungsmittel unter gleichzeitiger azeotroper Destillation des bei der Umsetzung entstehenden Wasser durchführt.

2. Verfahren gemäß Anspruch 1, dadurch gekennzeichnet, daß man die Umsetzung in Gegenwart von 1 bis 50 Gew.%, bezogen auf das Pyrazolon der Formel III, an wasserfreiem Natrium- oder Kaliumcarbonat durchführt.

3. Verfahren gemäß Anspruch 1, dadurch gekennzeichnet, daß man die Umsetzung in Xylol, Dichlorbenzol, Nitrobenzol oder Benzoesäuremethylester als Lösungsmittel durchführt.

## Claims

1. A process for preparing a pyrazolo[5,1-b]quinazolone of the formula I

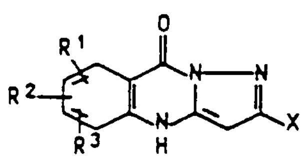

(I),

where $R^1$, $R^2$ and $R^3$ are identical or different and each is independently of the others hydrogen, halogen, $C_1$-$C_4$-alkoxy, nitro, $C_1$-$C_4$-alkyl, trifluoromethyl, carboxyl, cyano, carbamoyl, $C_1$-$C_4$-monoalkyl- or -dialkyl-carbamoyl, phenyl-carbamoyl, sulfamoyl, $C_1$-$C_4$-monoalkyl- or dialkylsulfamoyl, phenylsulfamoyl, hydroxysulfonyl or $C_1$-$C_4$-alkanoylaminoand X is $C_1$-$C_4$-alkyl or unsubstituted or $C_1$-$C_4$-alkyl-, $C_1$-$C_4$-alkoxy-, halogen-, nitro- or cyano-substituted phenyl, by reacting an isatoic anhydride of the

formula II

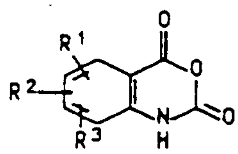

(II),

where $R^1$, $R^2$ and $R^3$ are each as defined above, with a pyrazolone of the formula III

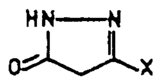

(III),

where X is as defined above, which comprises performing the reaction at from 100 to 180 °C in an inert organic solvent with simultaneous azeotropic distillation of the water formed in the course of the reaction.

**2.** A process as claimed in claim 1, wherein the reaction is carried out in the presence of from 1 to 50% by weight, based on the pyrazolone of the formula III, of anhydrous sodium carbonate or potassium carbonate.

**3.** A process as claimed in claim 1, wherein the reaction is carried out in xylene, dichlorobenzene, nitrobenzene or methyl benzoate as solvent.

**Revendications**

**1.** Procédé de préparation de pyrazolo[5,1-b]quinazolones de formule I

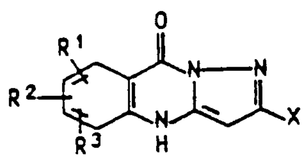

(I),

dans laquelle $R^1$, $R^2$ et $R^3$ sont identiques ou différents et représentent chacun indépendamment l'un de l'autre un atome d'hydrogène, d'halogène, un groupe alcoxy en $C_1$-$C_4$, nitro, alkyle en $C_1$-$C_4$, trifluorométhyle, carboxy, cyano, carbamoyle, mono- ou di(alkyle en $C_1$-$C_4$)carbamoyle, phénylcarbamoyle, sulfamoyle, mono- ou di(alkyle en $C_1$-$C_4$)sulfamoyle, phénylsulfamoyle, hydroxysulfonyle ou alcanoylamino en $C_1$-$C_4$ et X représente un groupe alkyle en $C_1$-$C_4$ ou phényle éventuellement substitue par un groupe alkyle en $C_1$-$C_4$, alcoxy en $C_1$-$C_4$, halogeno, nitro ou cyano, par réaction d'anhydrides isatoïques de formule II

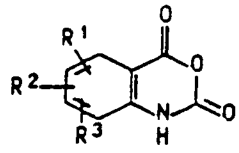

(II),

dans laquelle $R^1$, $R^2$ et $R^3$ ont chacun la signification mentionnée ci-dessus, avec des pyrazolones de formule III

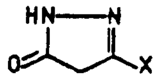

(III),

dans laquelle X a la signification mentionnée ci-dessus, caractérisé en ce que l'on effectue la réaction à une température de 100 à 180°C dans un solvant organique inerte sous distillation azéotrope simultanée de l'eau se formant au cours de la réaction.

**2.** Procédé selon la revendication 1, caractérisé en ce que l'on effectue la réaction en présence de 1 à 50% en poids, par rapport à la pyrazolone de formule III, de carbonate de sodium ou de potassium anhydre.

**3.** Procédé selon la revendication 1, caractérisé en ce que l'on effectue la réaction dans du xylène, du dichlorobenzène, du nitrobenzène ou du benzoate de méthyle comme solvant.